# EUROPÄISCHE PATENTANMELDUNG

(11) **EP 4 062 960 A1**
(43) Veröffentlichungstag der Anmeldung: **28.09.2022**
(21) Anmeldenummer: 21164568.4
(22) Anmeldetag: 24.03.2021
(51) Int. Cl.: A61M 16/04, A61M 16/10

(54) **ANORDNUNG IN ART EINES SPRECHVENTILS ZUM AUFSETZEN UND ANBRINGEN AN EINE TRACHEOSTOMIEKANÜLE**

(71) Anmelder: Albert-Ludwigs-Universität Freiburg, 79098 Freiburg (DE)
(72) Erfinder: Stahl, Dr. Claudius, 79194 Heuweiler (DE); Lücking, Dr. Klaus-Michael, 79100 Freiburg (DE); Tauber, Dr. Falk, 79110 Freiburg (DE); Speck, Prof. Dr. Thomas, 79227 Schallstadt (DE); Philipp, Auth, 79115 Freiburg (DE)
(74) Vertreter: Rösler, Uwe

(57) **Zusammenfassung**

Beschrieben wird eine Anordnung in Art eines Sprechventils zum Aufsetzen und Anbringen an eine Tracheostomiekanüle, mit einem einen Durchströmungskanal umschliessenden Durchströmungskörper, der einseitig endseitig eine geeignet ausgebildete Verbindungsstruktur zur Fügung an ein proximales Ende der Tracheostomiekanüle aufweist, an dessen der Verbindungsstruktur längs des Durchströmungskanals gegenüberliegenden Ende ein Einwegventil angeordnet ist und der zwischen der Verbindungsstruktur und dem Einwegventil eine durch eine Überdruckventilanordnung abschließbare Ventilöffnung vorsieht.

Die Erfindung zeichnet sich dadurch aus, dass die Überdruckventilanordnung wenigstens eine elastisch verformbare Ventilklappe aufweist, die in einem ersten Zustand die zwischen der Verbindungsstruktur und dem Einwegventil am Durchströmungskörper vorgesehene Ventilöffnung abzudecken vermag und bei einem vorgebbaren Überdruck innerhalb des Durchströmungskanals durch eine Überdruck getriebene, elastische Verformung der Ventilklappe in einen die Ventilöffnung zumindest teilweise freigebenden zweiten Zustand überführbar ist. Alternativ oder in Kombination ist mittel- oder unmittelbar an der Überdruckventilanordnung oder stromab zur Überdruckventilanordnung ein Signalerzeugungsmittel angeordnet, das im Falle eines Überdruck-getriebenen Öffnens der Überdruckventilanordnung ein Signal erzeugt.

## Beschreibung

### Technisches Gebiet

Die Erfindung bezieht sich auf eine Anordnung in Art eines Sprechventils zum Aufsetzen und Anbringen an eine Tracheostomiekanüle, mit einem einen Durchströmungskanal umschließenden Durchströmungskörper, der einseitig endseitig eine geeignet ausgebildete Verbindungsstruktur zur Fügung an ein proximales Ende der Tracheostomiekanüle aufweist, an dessen der Verbindungsstruktur längs des Durchströmungskanals gegenüberliegenden Ende ein Einwegventil angeordnet ist und der zwischen der Verbindungsstruktur und dem Einwegventil eine durch eine Überdruckventilanordnung abschließbare Ventilöffnung vorsieht.

Tracheostomiekanülen dienen dem direkten Zugang zur Luftröhre einer Person unter Umgehung des Mund-, Rachen- sowie Kehlkopfbereiches zum Zwecke einer direkten Luftzu- und -abführung in bzw. aus der Lunge. Neben sogenannten ungeblockten Tracheostomiekanülen, die vorwiegend bei spontan atmenden Patienten mit konstantem, reflektorischen und aspirationsfreiem Schlucken eingesetzt werden, dienen sogenannte geblockte Tracheostomiekanülen, die distalseitig einen dillatierfähigen Cuff vorsehen, zur invasiven Beatmung und/oder der Beatmung aspirationsgefährdeter Patienten, bei denen sicherzustellen ist, dass ein unkontrolliertes Entweichen von Atemgasen unter Beatmung durch den Rachen und Mundraum vermieden wird und zudem zu verhindern ist, dass insbesondere bei Patienten mit Schluckstörungen Speisereste oder Speichel ungehindert in die unteren Luftwege oder in die Lunge geraten.

Das offen ausgebildete, proximale Ende einer Tracheostomiekanüle verfügt über einen geeignet ausgebildeten Anschlussflansch zum Anbringen beispielsweise eines Beatmungsgerätes, einer Tubusverlängerung oder für den Aufsatz eines die Einatemluft wärmenden und befeuchtenden Durchströmungskörpers, kurz HME (Heat and Moisture-Exchanger) oder eines sogenannten Sprechventils.

Ein Sprechventil ermöglicht das Sprechen, sofern die Stimmbänder sowie die oberen Atemwege intakt und funktionsfähig sind. Ein Sprechventil ist ein Einwegventil, das so aufgebaut ist, dass es sich beim Einatmen öffnet und den Luftstrom über die Tracheostomiekanüle in die Lunge leitet und beim Ausatmen hingegen verschließt, so dass die Ausatemluft durch den Kehlkopf über die Stimmbänder zu deren Schallanregung geleitet wird. Hierzu ist es jedoch erforderlich, dass die Tracheostomiekanüle entblockt wird, d.h. der am distalen Ende der Tracheostomiekanüle angeordnete Cuff ist zu entleeren, so dass sich ein ausreichend großer Zwischenspalt zwischen Cuff und Luftröhre auszubilden vermag. Alternativ oder in Kombination zur Maßnahme der ungeblockten Tracheostomiekanüle unterstützen sogenannte gesiebte oder gefensterte Tracheostomiekanülen während der Ausatmung ein Überströmen der Stimmbänder mit Ausatemluft bei Verwendung eines Sprechventils.

Ein Problem entsteht jedoch bei der Verwendung eines Sprechventils mit einer nicht gefensterten Tracheostomiekanüle, wenn die Cuff versehentlich nicht entleert ist, d.h. die Tracheostomiekanüle befindet sich im geblockten Zustand, so dass es keinen Weg für die Ausatemluft des Patienten gibt. In diesem Fall erlaubt das Sprechventil zwar eine Einatmung, aber keine Ausatmung. Innerhalb weniger Atemzüge ist die Lunge maximal überbläht. Der Patient droht einerseits zu ersticken, da er nicht mehr atmen kann, und andererseits besteht die akute Gefahr eines Platzens der Lunge (Pneumothorax), etwa durch exzessiven Überdruck beim Husten.

Zu einer ähnlichen Situation kann es gelegentlich auch bei ungeblockter Tracheostomiekanüle kommen, wenn die Schleimhaut um die Tracheostomiekanüle unerwartet anschwillt und sich dadurch das Lumen zwischen Luftröhre und Tracheostomiekanüle verengt.

Zur Begegnung dieser Gefahren, die insbesondere ein Patient beim unmittelbaren Aufsetzen eines Sprechventils an einer geblockten und weder gefensterten noch gesiebten Tracheostomiekanüle ausgesetzt ist, wird in der Druckschrift WO 2016/139441 A1 vorgeschlagen, im Bereich des Sprechventilaufsatzes zwischen der Verbindung zur Tracheostomiekanüle und dem Einwegventil des Sprechventilsaufsatzes ein Überdruckventil anzuordnen, dessen Ventilfunktion derart eingestellt ist, dass das Überdruckventil bei für den Patienten ungefährlichen Druckverhältnissen innerhalb der Sprechventilanordnung fluid- bzw. gasdicht abschließt und ausschließlich bei Überschreiten eines vorgegebenen Überdruckes innerhalb der Sprechventilanordnung spontan auszulösen vermag, wodurch eine Ventilöffnung freigegeben wird, über die überschüssige Ausatemluftanteile in die Umgebung weitgehen widerstandsfrei abströmen kann.

Das bekannte Überdruckventil ist in Form eines Kugelventils ausgebildet, das aufgrund der Eigenschwere der Kugel eine Ventilöffnung gasdicht abzuschließen vermag. Größe, Gewicht und Anordnung der Kugel sind dabei derart bemessen, so dass im Falle einer für den Patienten gefährlichen Überdrucksituation die Kugel entgegen der Schwerkraft angehoben wird, wodurch die Entlüftungsöffnung freigegeben wird.

Auch das in der Druckschrift EP 2 908 895 B1 beschriebene Sprechventil für Tracheostomiekanülen verfügt neben der für Sprechventile charakteristischen Einwegventilfunktion über die Möglichkeit einer Entlüftung durch manuelles Überführen eines Öffnungsmechanismus in eine geöffnete Position, in der ein freies Atmen ermöglicht wird, wie es bei einer Tracheostomiekanüle ohne aufgebrachtes Sprechventil der Fall ist.

Der Druckschrift EP 2 326 376 B1 ist eine kombinierte Ventilanordnung zur Anbringung an eine Tracheostomiekanüle zu entnehmen, die zu Zwecken der künstlichen bzw. mechanischen Beatmung über ein Inspirationsventil sowie über ein getrennt dazu ausgebildetes Expirationsventil verfügt, wobei letzteres als einstellbares PEEP-Ventil (positiver Endex spiratorischer Druck) ausgebildet ist. Insbesondere handelt es sich bei dem Expirations-PEEP-Ventil um ein Kugel-Käfig-Ventil mit Feder, über deren Spannung der Luftstromwiderstand des Expirations-PEEP-Ventils einstellbar ist.

Trotz der auf dem Markt verfügbaren Sprechventilen sowie der Kenntnis und den informellen Hinweisen Sprechventilaufsätze nur auf ungeblockte Trachealkanülen aufzusetzen, kommt es weltweit nach wie vor zu Todesfällen bedingt durch das Aufsetzen eines Sprechventilaufsatzes auf eine geblockte Tracheostomiekanüle, und zwar auch dann, wenn erfahrene Operateure bzw. Betreuer die Kanüle aufsetzen. Das Bundesinstitut für Arzneimittel und Medizinprodukte hat infolgedessen im Juli 2019 auf diesen Gebrauchstauglichkeitsmangel verwiesen und eine bessere Kennzeichnung von Sprechventilaufsätzen verlangt. Selbst der Einsatz von als Kugelventile ausgebildeten Überdruckventilen im Bereich der Sprechventilanordnung kann funktionsbedingte Fehlfunktionen aufgrund ihrer räumlichen Lagesensitivität nicht ausschließen.

### Darstellung der Erfindung

Der Erfindung liegt die Aufgabe zugrunde eine Anordnung in Art eines Sprechventils zum Aufsetzen und Anbringen an eine Tracheostomiekanüle, mit einem einen Durchströmungskanal umschließenden Durchströmungskörper, der einseitig endseitig eine geeignet ausgebildete Verbindungsstruktur zur Fügung an ein proximales Ende der Tracheostomiekanüle aufweist, an dessen der Verbindungsstruktur längs des Durchströmungskanals gegenüber liegenden Ende ein Einwegventil angeordnet ist und zwischen der Verbindungsstruktur und dem Einwegventil eine durch eine Überdruckventilanordnung abschließbare Öffnung vorsieht, derart weiterzubilden, so dass die bislang bestehende Patientengefahr beim Aufsetzen eines Sprechventils auf bzw. an die Tracheostomiekanüle vermieden bzw. signifikant herabgesetzt werden kann. So soll ein vorstehend erläutertes Sprechventil zum einen sicherstellen, dass ein sich spontan aufbauender Überdruck im Luftweg eines Patienten, bspw. durch Husten, sicher und unverzüglich abgebaut wird und dies in jeder erdenklichen Raumlage der Sprechventilanordnung, d.h. unabhängig von der Einwirkungsrichtung der Schwerkraft. Alternativ oder in Kombination mit der vorstehenden Vorkehrung gilt es die Sprechventilanordnung dahingehend zu modifizieren bzw. zu ergänzen, so dass bei Auftritt einer lebensbedrohlichen Überdrucksituation, die zumindest zu einer Teilöffnung einer vorhandenen Überdruckventilanordnung führt, eine mit der Sprechventilanordnung befasste, betreuende Person über die ansonsten lebensbedrohliche Situation für den Patienten alarmiert wird.

Die Lösung der der Erfindung zugrunde liegenden Aufgabe ist in den Ansprüchen 1 und 2 angegeben. Den Erfindungsgedanken in vorteilhafter Weise weiterbildende Merkmale sind Gegenstand der Unteransprüche sowie der weiteren Beschreibung, insbesondere unter Bezugnahme auf die Ausführungsbeispiele zu entnehmen. Lösungsgemäß ist eine Anordnung in Art eines Sprechventils zum Aufsetzen und Anbringen an eine Tracheostomiekanüle mit den Merkmalen des Oberbegriffes des Anspruches 1 derart ausgebildet, dass die Überdruckventilanordnung wenigstens eine elastisch verformbare Ventilklappe aufweist, die in einem ersten Zustand (Druck im Sprechbereich) die zwischen der Verbindungsstruktur und dem Einwegventil am Durchströmungskörper vorgesehene Ventilöffnung abzudecken vermag und bei einem vorgebbaren Überdruck innerhalb des Durchströmungskanals durch eine Überdruck getriebene, elastische Verformung der Ventilklappe in einen die Ventilöffnung zumindest teilweise freigebenden zweiten Zustand (Überdruckbereich) überführbar ist.

Das Konstruktions- bzw. Funktionsprinzip der am Durchströmungskörper des Sprechventils angebrachten Überdruckventilanordnung ist inspiriert durch einen in der Natur vorkommenden Öffnungsmechanismus an einer bei der fleischfressenden Pflanze "Wasserschlauch" (*Utricularia vulgaris* / *Utricularia australis)* genutzten Saugfalle. Bei der Saugfalle handelt es sich um eine mit einer flexiblen Klappe verschließbaren Fangblase, die in der Lage ist, Flüssigkeit in Form von Wasser aus dem Fangblaseninneren via Drüsen in die Umgebung zu transportieren, wodurch sich die Fangblasenwände unter Ausbildung einer elastischen Wandspannung nach innen wölben, wobei der Falleneingang durch eine in diesem Zustand nach außen gewölbte Falltür, die mit ihrem freien Rand einer gewebigen Schwelle anliegt, wasserdicht verschlossen ist. In diesem Zustand herrscht im Falleninneren gegenüber dem Umgebungsdruck ein Unterdruck. Bei Kontakt mit einer Beute kehrt die Falltür ihre konvexe Krümmung innerhalb von weniger als 2 ms in eine konkave Form um, wodurch der Falleneingang zumindest bereichsweise freigegeben wird. In diesem unverschlossenen Saugfallenzustand erfolgt ein Unterdruck-getriebener Druckausgleich, wodurch sich die Saugfallenwände entspannen und Wasser sowie Beute unmittelbar vor der Saugfallenöffnung aufgrund der sich innerhalb einer Millisekunde zutragenden Volumenvergrößerung in das Innere der Saugfalle einströmen.

Die vorstehend erläuterte Funktionalität macht sich die in der Anordnung innerhalb eines Sprechventils integrierte Überdruckventilanordnung in abstrahierter (bzgl. der Druckverhältnisse umgekehrter) Weise zunutze, indem die wenigstens eine elastisch verformbare Ventilklappe, gleichsam der Fallentür im biologischen Vorbild entsprechend, ab einem schlagartig auftretenden Druckunterschied zwischen dem Inneren des Durchströmungskörpers, d.h. der Sprechventilanordnung und dem Umgebungsdruck, der üblicherweise dem aktuell vorherrschenden Luftdruck entspricht, den die Ventilöffnung zumindest teilweise freigebenden zweiten Zustand einnimmt, wodurch der Überdruck instantan abgebaut wird.

Um die Funktionalität des Sprechventils im Sprechbereich nicht zu beeinträchtigen, ist die Überdruck-abhängige Öffnung der Überdruckventilanordnung, die durch eine weitgehend verzögerungsfreie Überführung der wenigstens einen elastisch verformbaren Ventilklappe aus dem ersten in den zweiten Zustand realisiert wird, ab einem einstellbaren Druckunterschied, z.B. ab 35 bis 45 cm H2O, d.h. 0,034 bar bis 0,044 bar, zu initiieren. Im Druckbereich unterhalb des vorstehenden Auslösedruckes für die Überdruckventilanordnung, d.h. für Drucke im Sprechbereich zwischen 0 bis 25 cm H2O, d.h. 0 bis 0,0245 bar, verbleibt die wenigstens eine elastisch verformbare Ventilklappe im ersten, d.h. geschlossenen Zustand, so dass eine ausreichend druckbeaufschlagte Luftströmung im Normalbetrieb des Sprechventils die Patienten eigenen Stimmbänder zum Sprechen anzuregen vermag. Die tatsächlichen anzuwendenden Druckbereiche und die maximale Öffnungsfläche der Ventilklappe können je nach Muskelkraft, Größe und Lungenvolumen des Patienten unterschiedlich realisiert werden, indem vorzugsweise bei schwachen oder kleinen Patienten mit niedrigem Lungenvolumen der Überdruckbereich niedriger und die maximale Öffnungsfläche kleiner gewählt wird als bei kräftigen oder großen Patienten mit hohem Lungenvolumen.

Im Unterschied zu einer bekannten Kugelventilanordnung, deren Funktionsweise durch die Eigenschwere der Kugel und damit verbunden durch die räumliche Anordnung und Lage des gesamten Kugelventils abhängt, spielt die Eigenschwere der wenigstens einen elastisch verformbaren Ventilklappe für die Funktionsweise der lösungsgemäßen Überdruckventilanordnung keine bzw. keine nennenswerte Rolle, wodurch die Funktionsweise der lösungsgemäßen Anordnung innerhalb des Sprechventils in jeder räumlichen Anordnung und Lage gewährleistet bleibt.

In einer bevorzugten Ausführungsform ist eine einzige elastisch verformbare, flächig ausgebildete Ventilklappe vorgesehen, die im ersten Zustand die Ventilöffnung abdeckt, wobei die elastisch verformbare Ventilklappe relativ zur Ventilöffnung derart mittel- oder unmittelbar an den Durchströmungskörper gefügt ist, so dass die elastisch verformbare Ventilklappe den Durchströmungskanal zu- und der Ventilöffnung abgewandt angeordnet ist. Hierbei sind der Ventilöffnung zwei Halbräume zuordenbar, von denen in einem der beiden Halbräume die elastisch verformbare Ventilklappe wenigstens abschnittsweise ortsfest relativ zur Ventilöffnung gefügt ist und innerhalb des anderen der beiden Halbräume die elastisch verformbare Ventilklappe lose zur Ventilöffnung gelagert ist. Im übertragenen Sinne entspricht die Fügung der elastisch verformbaren Ventilklappe relativ zur Ventilöffnung einer einseitig an einer Türzarge drehbeweglichen Anbringung einer Schwenktür. Im Unterschied zur Anbringung und Ausbildung einer an sich bekannten Schwenktür deckt die wenigstens eine elastisch verformbare Ventilklappe im ersten Zustand die Ventilöffnung innerhalb ihres zweiten Halbraumes zumindest abschnittsweise mit einem randseitigen Übermaß ab, d.h. die elastisch verformbare Ventilklappe erstreckt sich zumindest abschnittsweise über einen Bereich des Ventilöffnungsrandes hinaus. Dieses randseitige Übermaß trägt dazu bei, dass die elastisch verformbare Ventilklappe bei einem in Betrieb befindlichen Sprechventil so lange innerhalb des ersten Zustandes, d.h. in dem die Ventilöffnung verschließenden Zustand verbleibt, solange der innerhalb der Sprechventilanordnung vorherrschende Druck unterhalb des vorgegebenen Überdruckbereichs, verbleibt.

Neben der Dimensionierung des Übermaßes spielen eine Vielzahl weiterer Material- und Designparameter eine wichtige Rolle zur Einstellung der Überdruck-getriebenen Öffnungsfunktion der neuartig ausgebildeten Überdruckventilanordnung. Wie im weiteren unter Bezugnahme auf ein illustriertes Ausführungsbeispiel erläutert wird, tragen Formgebung, Größe und Materialwahl für die Ausbildung der wenigstens einen elastisch verformbaren Ventilklappe sowie auch die Geometrie der randseitigen Ventilöffnungskontur für eine sichere und zuverlässige Funktion der Überdruckventilanordnung bei.

Vorzugsweise ist das randseitige Übermaß nicht gleich bemessen, sondern weist an wenigstens einer Stelle ein Minimum auf. Auf diese Weise lässt sich der Öffnungsvorgang durch die Überdruck-getriebene elastische Verformung der wenigstens eine Ventilklappe in einer geometrisch vorbestimmten Weise festlegen, in der die wenigstens eine Ventilklappe zeitlich am schnellsten eine elastische Umformung vom ersten in den zweiten Zustand realisiert.

Aufgrund der typischerweise zylinderförmigen Ausgestaltung des Durchströmungskörpers an sich bekannter Sprechventilaufsätze auf Tracheostomiekanülen bietet es sich an, die Ventilöffnung zwischen der Verbindungsstruktur und dem Einwegventil durch eine rechteckförmige oder schlitzförmige Ausnehmung innerhalb der Durchströmungskörperwand auszubilden oder durch eine Ventilöffnung, die sich an der rechteckförmigen oder schlitzförmigen Ausnehmung innerhalb der Durchströmungskörperwand anschließt und deren größere Öffnungsdimension in Umfangsrichtung um den Durchströmungskanal und deren kleinere Öffnungsdimension parallel zum Durchströmungskanal angeordnet sind.

Dabei ist die wenigstens eine elastisch verformbare Ventilklappe flächig ausgebildet und verfügt über eine flächig gebogene Form entsprechend angepasst zur Abdeckung der Ventilöffnung.

Um dem bereits erwähnten Aspekt der unterschiedlichen Sprechdrucke bei Patienten unterschiedlicher Konstitution Rechnung zu tragen, ist die wenigstens eine elastisch verformbare Ventilklappe austauschbar mittel- oder unmittelbar am Durchströmungskörper der Anordnung in Art eines Sprechventils anbringbar. Zur lösbar einseitig festen Anbringung der elastisch verformbaren Ventilklappe dienen zwei Befestigungspins, an denen die Ventilklappe unter Ausbildung einer flächig in sich gebogenen Raumform aufsteckbar ist. Durch Bevorratung einer Anzahl von Ventilklappen, die sich wenigstens durch ihre Form, Größe oder ihren elastischen Eigenschaften voneinander unterscheiden, kann eine patientenindividuelle Auswahl getroffen werden, um den Auslösedruck der Überdruckventilanordnung Patientenspezifisch wählen zu können. Nähere Einzelheiten können im Weiteren der Beschreibung unter Bezugnahme auf ein illustriertes Ausführungsbeispiel entnommen werden.

Alternativ oder in Kombination zur Ausbildung der vorstehend erläuterten sicher und Lage-unabhängig öffnenden Überdruckventilanordnung lässt sich die bei Fehlbedienung einer Sprechventilanordnung entstehende Lebensgefahr für den Patienten dadurch vermeiden bzw. signifikant reduzieren, indem mittel- oder unmittelbar an der Überdruckventilanordnung oder stromab zu dieser, wie auch immer die Überdruckventilanordnung ausgebildet sein mag, ein Signalerzeugungsmittel angeordnet ist, das im Falle eines Überdruck-getriebenen Öffnens der Überdruckventilanordnung, d.h. im Überdruckbereich, ein Signal erzeugt.

Vorzugsweise ist das Signalerzeugungsmittel zur Erzeugung eines akustisch wahrnehmbaren Schallsignals ausgebildet, das von einer die Sprechventilanordnung handhabenden Person in Form eines Alarmsignals unüberhörbar wahrgenommen werden kann. Durch das Alarmsignal wird die Person auf einen etwaigen Fehler aufmerksam gemacht, so dass der Fehler einer geblockten Tracheostomiekanüle unverzüglich korrigiert werden kann.

Gleichzeitig zur Signalerzeugung erfolgt eine spontane Druckentlastung durch die geöffnete Überdruckventilanordnung, wodurch die Gefahr des sofortigen Erstickens und Platzens der Patientenlunge verhindert wird. Der den Sprechventilaufsatz handhabenden Person wird dadurch Zeit gegeben, den Bedienfehler zu korrigieren. Durch die lösungsgemäße Kombination einer Signalerzeugung mit der Funktion der Druckentlastung, vorzugsweise auf Basis der vorstehend erläuterten, lösungsgemäß ausgebildeten Überdruckventilanordnung, werden einerseits die ungestörte Funktion des Sprechventils im normalen Einsatz, d.h. bei normalen Atmen und Sprechen vorherrschenden Drucken (Sprechbereich), ohne Fehlalarme gewährleistet und andererseits bei Überschreiten eines vorgegebenen Überdruckbereichs innerhalb des Sprechventilaufsatzes sowohl eine Signalauslösung als auch eine unverzügliche Druckentlastung zum Schutze des Patienten gleichzeitig realisiert.

In einer bevorzugten Ausführungsform ist das Signalerzeugungsmittel in Art einer Pfeife ausgebildet und befindet sich innerhalb eines sich stromab an die Überdruckventilanordnung anschließenden Strömungskanals. Je nach Ausbildung und Anordnung der Pfeife bietet es sich an, den Strömungskanal unter Ausbildung einer Labial- oder Lingualpfeife lokal zu strukturieren.

Alternativ oder in Kombination vermag das Signalerzeugungsmittel ein elektrisches, elektromagnetisches oder optisches Signal zu erzeugen, das entweder in unmittelbarer Umgebung zur physiologischen Wahrnehmung durch eine Person abgegeben wird oder mittels Kabel bzw. kabellos an eine Überwachungseinheit, beispielsweise in Form einer zentralen Monitoreinheit, übertragen wird. Hierzu ist eine die Überdrucksituation erfassende Sensorik in oder an die Überdruckventilanordnung anzuordnen, deren Sensorsignale einer zentralen Überwachungseinheit zur Alarmauslösung zuführbar oder in eine physiologisch wahrnehmbare Form transformierbar sind. Die Sensorik kann dabei mit dem Ventilmechanismus kombiniert sein oder als unabhängiger Signalgeber realisiert werden.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnung exemplarisch beschrieben. Es zeigen:
- Fig. 1: perspektivische Längsschnittdarstellung durch eine lösungsgemäß ausgebildete Anordnung in Art eines Sprechventils,
- Fig. 2a-f: Querschnittsdarstellung einer lösungsgemäßen Ausbildung einer Überdruckventilanordnung als Sequenzbilder,
- Fig. 3a-j: perspektivische Längsschnittbilder durch eine lösungsgemäß ausgebildete Anordnung in Art eines Sprechventils als Sequenzbilder sowie
- Fig. 4: perspektivische Querschnittsdarstellung durch eine lösungsgemäß ausgebildete Anordnung in Art eines Sprechventils.

### Wege zur Ausführung der Erfindung, gewerbliche Verwendbarkeit

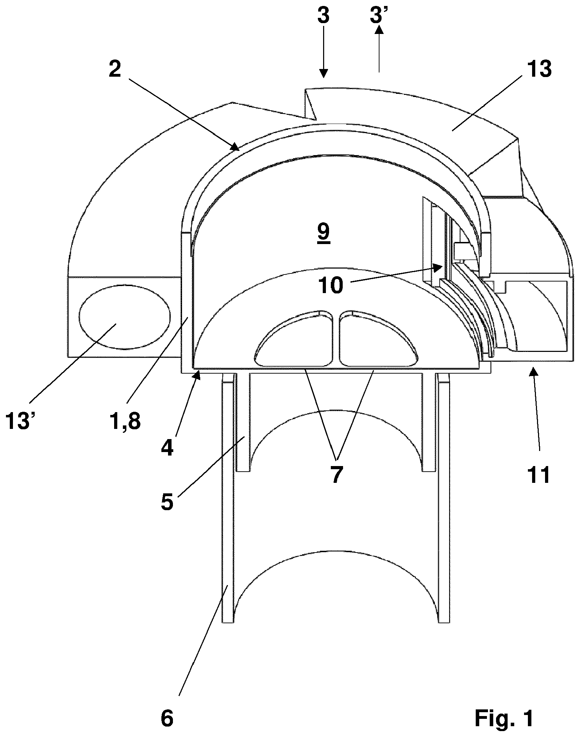

Figur 1 zeigt eine perspektivische Längsschnittdarstellung durch eine Sprechventilanordnung, die über einen hohlzylinderförmigen Durchströmungskörper 1 verfügt, an dessen oberen, in Figur 1 aus besseren Darstellungsgründen offen ausgebildeten Durchströmungskörperende 2 ein nicht weiter, an sich bekanntes Einwegmembranklappenventil angeordnet ist, das das obere Durchströmungskörperende für eine inspiratorische Luftzuströmung 3 öffnet und eine respiratorische Luftabströmung 3' unterbindet.

An dem in Figur 1 unteren Durchströmungskörperende 4 schließt eine Verbindungsstruktur 5 an, über die die Sprechventilanordnung vorzugsweise abnehmbar fluiddicht an eine Tracheostomiekanüle 6 fügbar ist. Am Übergang zwischen Durchströmungskörper 1 und Verbindungsstruktur 5, die im gezeigten Ausführungsbeispiel über einen geringeren Innendurchmesser verfügt als der Durchströmungskörper 1, ist eine Gitterstruktur 7 angeordnet, die zum einen eine Schutzfunktion gegen unkontrollierten Eintritt von Fremdpartikeln in Richtung der Tracheostomiekanüle 6 besitzt und zum anderen eine mechanisch stabile Auflageebene für ein nicht dargestelltes, standardmäßiges Partikelfilterschaumelement bietet.

Zusätzlich sieht die Anordnung in Art eines Sprechventils innerhalb des Durchströmungskörpers 1, dessen Durchströmungskörperwand 8 einen Durchströmungskanal 9 umfasst, eine Öffnung 10 vor, die die Durchströmungskörperwand 8 vollständig durchsetzt und zwischen dem oberen Durchströmungskörperende 2 und dem unteren Durchströmungskörperende 4 angeordnet ist.

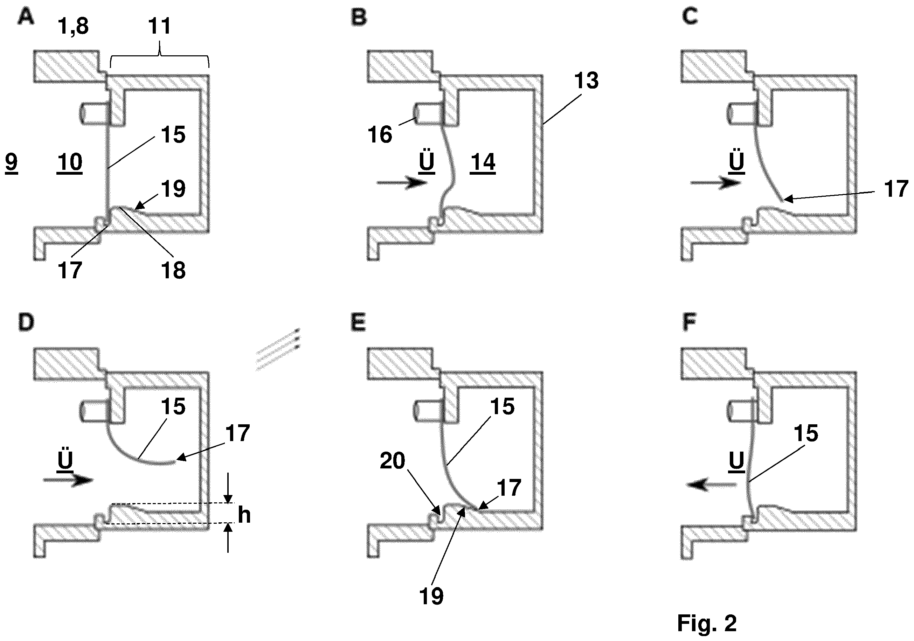

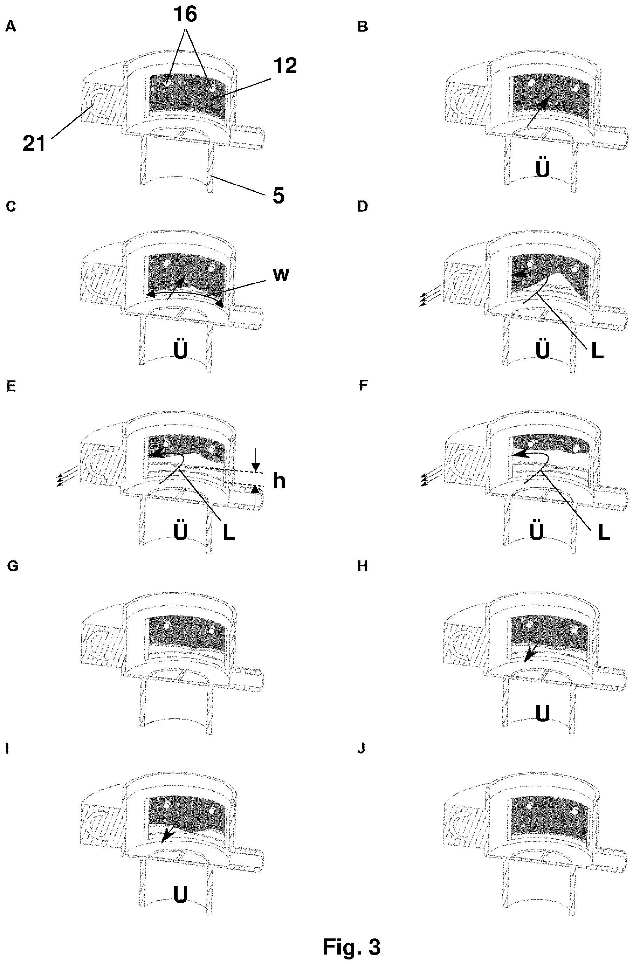

An der Öffnung 10 ist eine Überdruckventilanordnung 11 angebracht, deren Aufbau und Funktionsweise unter Bezugnahme der in den Figuren 2 und 3 entnehmbaren Sequenzbildern im Weiteren näher erläutert wird.

Die Sequenzbilder der Figuren 2a bis f stellen jeweils Längsschnitte durch die an der Öffnung 10 innerhalb der Durchströmungskörperwand 8 des Durchströmungskörpers 1 angeordneten Überdruckventilanordnung 11 dar. Die Durchströmungskörperwand 8 umfasst radial den Durchströmungskanal 9. Die in den Figuren 3a bis j gezeigten Sequenzbilder stellen perspektivische Längsschnitte durch die gesamte Anordnung in Art eines Sprechventils dar. Sämtliche in den Figuren 2 und 3 illustrierten Sequenzbilder vermögen die Funktionsweise und das dynamische Verformungsverhalten der die Überdruckventilfunktion bestimmenden elastisch verformbaren Ventilklappe 12 nachvollziehbar zu charakterisieren.

Die Überdruckventilanordnung 11 schließt sich unmittelbar radial außerhalb des Durchströmungskörpers 1 an die Öffnung 10 innerhalb der Durchströmungskörperwand 8 fluiddicht an. Die Überdruckventilanordnung 11 kann als separate Baueinheit zum Durchströmungskörper 1, wie dargestellt, ausgebildet sein oder einstückig mit diesem verbunden sein. Die Überdruckventilanordnung 11 verfügt über eine Ventilöffnung 12, die im dargestellten Fall kleiner dimensioniert ist als die Öffnung 10. Denkbar sind jedoch auch Ausführungsvarianten für die Überdruckventilanordnung 11, in denen die Öffnung 10 sowie die Ventilöffnung 12 identisch sind.

Die Überdruckventilanordnung 11 weist eine, einen Strömungskanal 14 umfassende Strömungskanalwand 13 auf, die über wenigstens eine Öffnung 13' in die Umgebung mündet, s. Fig. 1. Somit herrschen innerhalb des Strömungskanals 14 Umgebungsdruckbedingungen.

Die Ventilöffnung 12 ist in einem ersten Zustand, siehe Figur 2a, 3a von einer elastisch verformbaren Ventilklappe 15 vollständig abgedeckt. Die Ventilklappe 15 ist membran- bzw. folienartig ausgebildet und besteht bspw. aus einem Elastomer. Zur Anbringung der Ventilklappe 15 innerhalb der Überdruckventilanordnung 11 dienen zwei Befestigungspins 16 die in radialer Projektion auf die Öffnung 10 innerhalb des Öffnungsbereiches der Öffnung 10 an der Überdruckventilanordnung 11 angebracht sind, siehe insbesondere die Darstellung gemäß Figur 3a bis j. Die elastisch verformbare Ventilklappe 15 weist zu den Befestigungspins 16 korrespondierende Löcher auf, so dass die Ventilklappe 15 längs ihres oberen Ventilklappenrandes abnehmbar fest an der Überdruckventilanordnung 11 gefügt ist.

Form und Größe der elastisch verformbaren Ventilklappe 15 sind an die Form und Größe der Ventilöffnung 12 derart angepasst, so dass sie axial beiderseits die Ventilöffnung 12 mit einem Übermaß überragt, wohingegen die Erstreckung der Ventilklappe 15 in Umfangsrichtung maximal der Ventilöffnungsweite w der Ventilöffnung 12 entspricht, s. Figur 3c. Die Fügung der elastisch verformbaren Ventilklappe 15 an die Überdruckventilanordnung 11 gestattet überdies einen unkomplizierten Austausch der Ventilklappe 15.

In einem nicht gefügten Zustand ist die elastisch verformbare Ventilklappe 15 flächig rechteckförmig eben ausgebildet. Nach Fügen der Ventilklappe 15 in die Überdruckventilanordnung 11 mittels der Befestigungspins 16 wird die Ventilklappe 15 in einen flächig gebogenen, entsprechend angepasst an die zylindrische Krümmung der Durchströmungskörperwand 8 überführt, wodurch die elastisch verformbare Ventilklappe 15 eine durch die Biegung, deren Größe, Form und Eigenelastizität des Ventilklappenmaterials mitbestimmte Formstabilität erhält.

Der den Befestigungspins 16 axial gegenüberliegende untere Ventilklappenrand 17 der Ventilklappe 15 überragt axial den die Ventilöffnung 12 begrenzenden unteren Rand, der schwellenartig ausgebildet ist, siehe hierzu Figuren 2a bis f. Die schwellenartige Ausbildung des unteren Ventilöffnungsrandes 18 sieht eine der Ventilklappe 15 im ersten Zustand zugewandte axial orientierte Flanke vor, an der der untere Ventilklappenrand 17 mit Übermaß bündig anliegt. In Richtung des inneren Strömungskanals 14 der Überdruckventilanordnung 11 schließt sich eine kontinuierlich abfallende Schwellenflanke 19 an, deren Funktion im Weiteren noch erläutert wird. Zudem verfügt der schwellenförmig ausgebildete untere Ventilöffnungsrand 18 in Umfangsrichtung der Überdruckventilanordnung 11 über eine variierende Schwellenhöhe h dergestalt, dass die Schwellenhöhe h jeweils an beiden sich in Umfangsrichtung gegenüberliegenden Ventilöffnungsrändern maximal und jeweils mittig minimal ist, siehe Figuren 2d, 3e,f. Auf diese Weise überlappt die elastisch verformbare Ventilklappe 15 die Ventilöffnung 12 jeweils mit einem maximalen Übermaß an ihren Randbereichen und mit einem minimalen Übermaß im mittigen Bereich der Ventilklappe 15.

Die Form, Größe, Dicke sowie das elastomere Material der elastisch verformbaren Ventilklappe 15 bestimmen das elastisch Verformungsverhalten der Ventilklappe und legen somit den für das Öffnen der Überdruckventilanordnung erforderlichen Mindestüberdruck fest. Durch Vorsehen einer Anzahl unterschiedlich konfektionierter Ventilklappen, die sich in ihren elastischen Verformungsverhalten voneinander unterscheiden, kann die lösungsgemäße Anordnung in Art eines Sprechventils an die Beatmungsphysiologischen Fähigkeiten bzw. Eigenschaften eines Patienten individuell angepasst werden.

Im Weiteren wird auf die Sequenzbilder sowohl der Figuren 2 und 3 gemeinsam Bezug genommen, um die Überführung der elastisch verformbaren Ventilklappe 15 aus dem ersten Zustand, in dem die Überdruckventilanordnung 11 geschlossen ist, in den zweiten Zustand, in dem die Überdruckventilanordnung 11 eine geöffnete Stellung einnimmt, näher zu erläutern.

Die Figuren 2a, 3b zeigen die Überdruckventilanordnung 11 im ersten Zustand, d.h. die Ventilklappe 15 schließt die Ventilöffnung 12 weitgehend gasdicht ab und sorgt dafür, dass sich innerhalb des Durchströmungskörpers 1 Druckverhältnisse ausbilden können, die dem Patienten ein Sprechen ermöglichen.

Erhöht sich der Druck innerhalb des Durchströmungskörpers 1, beispielsweise durch spontanes Husten, so wird die elastisch verformbare Ventilklappe 15 Überdruck-getrieben deformiert, siehe Figur 2b, 3b. Bei einem ausreichend hohen, innenseitig, d.h. von Seiten des Durchströmungskanals 9, auf die Ventilklappe 15 lastenden Überdruckes Ü wird die Ventilklappe 15 durch das Druckereignis vornehmlich im mittigen Bereich radial nach außen deformiert, so dass der untere frei bewegliche Ventilklappenrand 17 den unteren schwellenartig ausgebildeten Ventilöffnungsrand 18, der mittig gegenüber den Randbereichen abgesenkt ist, überstreift. In diesem sich öffnenden Moment der Überdruckventilanordnung 11 bildet sich aufgrund der schwellenartigen Geometrie längs des unteren Ventilöffnungsrandes 18 eine zentrale senkrechte Falz am unteren Ventilklappenrand 17 aus, siehe Figur 3c, wodurch sich die Ventilklappe 15 von ihrer Mitte ausgehend schlag- bzw. sprungartig radial nach außen in das Volumen der Überdruckventilanordnung 11 deformiert, siehe Figur 3d, 2c. In diesem Moment kommt es sowohl zu einer temporären Versteifung der Ventilklappe 15 in ihrem Zentrum als auch zu einer strukturellen Instabilität der Fläche der Ventilklappe 15 durch das Aufheben ihrer Flächenkrümmung über die gesamte Ventilklappe 15. Im Weiteren wird die Ventilklappe 15 Überdruck-getrieben von ihrer Mitte ausgehend in das Lumen, bzw. in den Strömungskanal 14 der Überdruckventilanordnung 11 verdrängt, wobei die randseitigen Bereiche der Ventilklappe vom unteren Ventilöffnungsrand 18 nachgezogen werden. Die Ventilöffnung 12 wird freigegeben, siehe die Figuren 2c, 2d bzw. 3e, f, wobei der sich schlagartig innerhalb des Durchströmungskörpers 1 ausgebildete Überdruck Ü durch eine durch die Ventilöffnung 12 gerichtete Luftströmung L effektiv abgebaut wird.

Durch Abbau des Überdruckes bzw. vollständigen Druckausgleich stellt sich die elastisch verformbare Ventilklappe aufgrund ihrer Eigenelastizität wieder zurück, siehe Figur 2e, 3g und kommt mit ihrem unteren Ventilklappenrand 17 zur Anlage an die schräg geneigte Schwellenflanke 19 des unteren Ventilöffnungsrandes 18, siehe Figur 2e. Erfolgt kein weiterer Druckstoß, ausgelöst durch Husten, wird die Ventilklappe 15, gegebenenfalls unterstützt durch einen sich innerhalb des Durchströmungskörpers 1 einatmungsbedingt ausbildenden Unterdrucks U in den ersten Zustand rückgeführt, in dem die Ventilklappe 15 die Ventilöffnung 12 vollständig abdeckt, siehe Figur 2f, 3j.

In einer bevorzugten Ausführungsform, wie sie in den Sequenzbilddarstellungen gemäß Figur 2a bis f gezeigt ist, mündet der untere Ventilklappenrand 17 in eine nutförmige Ausnehmung 20, wodurch zusätzlich zur krümmungsbedingten Eigenstabilität der Ventilklappe 15 im ersten Zustand die Bewegungsmöglichkeit der Ventilklappe 15 begrenzt ist. Sowohl durch die gebogene Form der Ventilklappe 15, die durch die zylindrische bzw. runde Form des Durchströmungskörpers bedingt ist, sowie durch ein auf der Gitterstruktur 7 aufliegenden Partikelfilterschaumelementes (nicht dargestellt) sowie durch die Fügung der Ventilkappe 15 innerhalb der nutförmigen Ausnehmung 20 wird ein Durchschlagen oder Einsaugen der Ventilklappe 15 in das Innere der Sprechventilanordnung verhindert.

Das aus dem Bereich der Biologie entlehnte Ventilklappenprinzip, wie eingangs erläutert, basiert ausschließlich auf einer isotrop wirkenden Druckdifferenz zwischen dem Inneren des Durchströmungskörpers 1 und der Umgebung. Somit ist die Funktionsweise der "bionisch angelehnten" Überdruckventilanordnung 11 lageunabhängig.

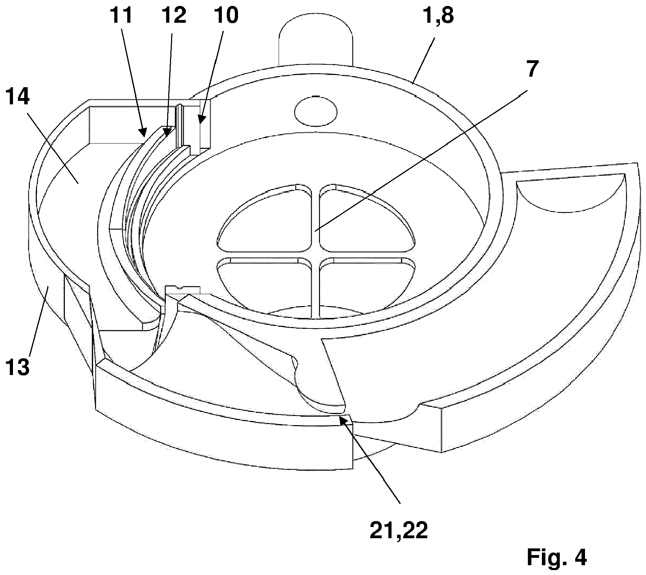

In einer bevorzugten Weiterbildung der Anordnung nach Art eines Sprechventils, zur Begegnung der Gefahr einer für den Patienten lebensbedrohlichen Situation durch unsachgemäße Handhabung der Tracheostomiekanüle, ist stromab zur Überdruckventilanordnung 11 ein Signalerzeugungsmittel 21 zur Erzeugung eines akustisch wahrnehmbaren Schallsignals, vorzugsweise in Form einer Pfeife 22, angeordnet, siehe hierzu Figur 3 sowie Figur 4. In Figur 4 ist der Durchströmungskörper 1 samt Überdruckventilanordnung 11 in einem Querschnitt dargestellt. Stromab zur Öffnung 10 bzw. Ventilöffnung 12 schließt sich ein Strömungskanal 14 an, der im Falle der in Figur 4 dargestellten Ausführungsform in Umfangsrichtung des Durchströmungskörpers 1 orientiert ist. Längs des Durchströmungskanals 12 ist eine Pfeife 21, vorzugsweise in Form einer Labial-Pfeifenkontur angeordnet. Kommt es zu einer Patienten-gefährdenden Überdrucksituation, so wird die Überdruckventilanordnung 11, beispielsweise in der vorstehend genannten Weise, schlagartig geöffnet, wodurch ein Entweichen der Überdruck-getriebenen Luftströmung durch die Überdruckventilanordnung 11 im Bereich der Pfeife 22 einen für das Umfeld alarmierenden akustisch wahrnehmbaren Signalton erzeugt, so dass eine den Patienten betreuende Person unverzüglich auf die Gefahrensituation aufmerksam gemacht wird und entsprechende Gegenmaßnahmen treffen kann.

Nicht notwendigerweise jedoch in vorteilhafter Form ist das Signalerzeugungsmittel 21 mit dem vorstehend erläuterten bionischen Überdruckventil in der in Figur 4 entnehmbaren Weise kombiniert. Gleichsam können an sich bekannte Überdruckventilanordnungen, beispielsweise in Form eines Kugelventils, im Bereich der Luftauslassöffnung mit einem entsprechenden Signalerzeugungsmittel, vorzugsweise in Form einer Pfeife, versehen sein.

Denkbar ist auch die Ausgestaltung des Signalerzeugungsmittels zur Erzeugung eines elektrischen, elektromagnetischen oder optischen Signals, das gegebenenfalls mittels Kabel oder kabellos an eine zentrale Überwachungseinheit übertragbar ist. Hierzu stehen dem Fachmann an sich bekannte Sensorsysteme zur Verfügung, mit denen bspw. eine Auslenkung der elastisch verformbaren Ventilkappe und/oder eine Luftströmung innerhalb des Strömungskanals detektierbar ist.

### Bezugszeichenliste

- 1: Durchströmungskörper
- 2: Durchströmungskörperende
- 3: Inspiratorische Luftzuströmung
- 3': Respiratorische Luftabströmung
- 4: Unteres Durchströmungskörperende
- 5: Verbindungsstruktur
- 6: Tracheostomiekanüle
- 7: Gitterstruktur
- 8: Durchströmungskörperwand
- 9: Durchströmungskanal
- 10: Öffnung
- 11: Überdruckventilanordnung
- 12: Ventilöffnung
- 13: Strömungskanalwand
- 14: Strömungskanal
- 15: Ventilklappe
- 16: Befestigungspin
- 17: Ventilklappenrand
- 18: Unterer Ventilöffnungsrand
- 19: Schwellenflanke
- 20: Nutförmige Ausnehmung
- 21: Signalerzeugungsmittel
- 22: Pfeife
- L: Luftströmung
- Ü: Überdruck
- U: Unterdruck, Einatmung

## Patentansprüche

1. Anordnung in Art eines Sprechventils zum Aufsetzen und Anbringen an eine Tracheostomiekanüle, mit einem einen Durchströmungskanal umschliessenden Durchströmungskörper, der einseitig endseitig eine geeignet ausgebildete Verbindungsstruktur zur Fügung an ein proximales Ende der Tracheostomiekanüle aufweist, an dessen der Verbindungsstruktur längs des Durchströmungskanals gegenüberliegenden Ende ein Einwegventil angeordnet ist und zwischen der Verbindungsstruktur und dem Einwegventil eine durch eine Überdruckventilanordnung abschließbare Ventilöffnung vorsieht,
**dadurch gekennzeichnet, dass** die Überdruckventilanordnung wenigstens eine elastisch verformbare Ventilklappe aufweist, die in einem ersten Zustand die zwischen der Verbindungsstruktur und dem Einwegventil am Durchströmungskörper vorgesehene Ventilöffnung abzudecken vermag und bei einem vorgebbaren Überdruck innerhalb des Durchströmungskanals durch eine Überdruck getriebene, elastische Verformung der Ventilklappe in einen die Ventilöffnung zumindest teilweise freigebenden zweiten Zustand überführbar ist.

2. Anordnung nach dem Oberbegriff des Anspruches 1 oder dem Anspruch 1, **dadurch gekennzeichnet, dass** mittel- oder unmittelbar an der Überdruckventilanordnung oder stromab zur Überdruckventilanordnung ein Signalerzeugungsmittel angeordnet ist, das im Falle eines Überdruck-getriebenen Öffnens der Überdruckventilanordnung ein Signal erzeugt.

3. Anordnung nach Anspruch 2,
**dadurch gekennzeichnet, dass** das Signalerzeugungsmittel zur Erzeugung eines akustisch wahrnehmbaren Schallsignals ausgebildet ist.

4. Anordnung nach Anspruch 3,
**dadurch gekennzeichnet, dass** das Signalerzeugungsmittel das Schallsignal durch Schwingungsanregung einer die Überdruckventilanordnung passierenden Luftströmung erzeugt.

5. Anordnung nach Anspruch 3 oder 4,
**dadurch gekennzeichnet, dass** sich stromab zu der durch die Überdruckventilanordnung abschließbaren Ventilöffnung ein Strömungskanal anschließt, längs dem das Signalerzeugungsmittel angeordnet ist.

6. Anordnung nach einem der Ansprüche 3 bis 5,
**dadurch gekennzeichnet, dass** das Signalerzeugungsmittel in Art einer Pfeife ausgebildet ist.

7. Anordnung nach Anspruch 6,
**dadurch gekennzeichnet, dass** die Pfeife in Art einer Labial- oder Lingualpfeife ausgebildet ist.

8. Anordnung nach einem der Ansprüche 2 oder 3,
**dadurch gekennzeichnet, dass** das Signalerzeugungsmittel ein elektrisches, elektromagnetisches oder optisches Signal zu erzeugen in der Lage ist, und
dass das Signalerzeugungsmittel mit einer Überwachungseinheit kabel- oder kabellos verbunden ist, an die das Signal übertragbar ist.

9. Anordnung nach einem der Ansprüche 5 bis 8,
**dadurch gekennzeichnet, dass** der Strömungskanal von einer Strömungskanalwand oder vom Durchströmungskörper und von einer Strömungskanalwand begrenzt ist.

10. Anordnung nach einem der Ansprüche 1 bis Anspruch 9,
**dadurch gekennzeichnet, dass** die wenigstens eine elastisch verformbare Ventilklappe über eine Form und Eigenelastizität verfügt, die eine Rückstellkraft basierte Wirkung entfalten, durch die die elastisch verformbare Ventilklappe aus dem zweiten Zustand in den ersten Zustand zumindest teilweise überführbar ist.

11. Anordnung nach einem der Ansprüche 1 bis 10,
**dadurch gekennzeichnet, dass** der Ventilöffnung zwei Halbräume zuordenbar sind,
dass die elastisch verformbare Ventilklappe innerhalb eines der beiden Halbräume wenigstens abschnittsweise ortsfest relativ zur Ventilöffnung gefügt ist, und dass die elastisch verformbare Ventilklappe in dem anderen der beiden Halbräume lose zur Ventilöffnung gelagert ist.

12. Anordnung nach Anspruch 11,
**dadurch gekennzeichnet, dass** die wenigstens eine elastisch verformbare Ventilklappe mittel- oder unmittelbar am Durchströmungskörper austauschbar gefügt ist.

13. Anordnung nach Anspruch 12,
**dadurch gekennzeichnet, dass** wenigstens zwei elastisch verformbare Ventilklappen bevorratet sind, die sich wenigstens in Form, Größe oder in ihren elastischen Eigenschaften voneinander unterscheiden, von denen wenigstens eine in Abhängigkeit Beatmungs-physiologischer Patienteneigenschaften mittel- oder unmittelbar am Durchströmungskörper fügbar ist.

14. Anordnung nach einem der Ansprüche 11 bis 13,
**dadurch gekennzeichnet, dass** innerhalb des zweiten Halbraumes die wenigstens eine elastisch verformbare Ventilklappe im ersten Zustand zumindest abschnittsweise mit einem randseitigen Übermaß die Ventilöffnung abdeckt, d.h. die Ventilklappe erstreckt sich zumindest abschnittsweise über die Ventilöffnungsweite hinaus.

15. Anordnung nach Anspruch 14,
**dadurch gekennzeichnet, dass** das randseitige Übermaß an wenigstens einer Stelle minimal ist.

16. Anordnung nach einem der Ansprüche 1 bis 15,
**dadurch gekennzeichnet, dass** die wenigstens eine elastisch verformbare Ventilklappe flächig ausgebildet und über eine gebogene Form verfügt.
